# EUROPEAN PATENT APPLICATION

(11) **EP 4 378 529 A1**
(43) Date of publication of application: **05.06.2024**
(21) Application number: 22211271.6
(22) Date of filing: 03.12.2022
(51) Int. Cl.: A61P 35/00, A61P 35/02, C07K 16/28, C07K 16/46

(54) **ANTIBODY FOR USE IN A THERAPY INVOLVING EFFECTOR CELL ENGAGEMENT**

(71) Applicant: Universitätsklinikum Hamburg-Eppendorf, 20246 Hamburg (DE)
(72) Inventor: Ayuk, Francis Ayuketang, 20246 Hamburg (DE); Fehse, Boris, 20246 Hamburg (DE); Riecken, Kristoffer, 20246 Hamburg (DE); Hambach, Julia, 20246 Hamburg (DE); Koch-Nolte, Friedrich, 20246 Hamburg (DE); Harfmann, Maraike, 24558 Henstedt-Ulzburg (DE); Möller, Constantin, 20246 Hamburg (DE)
(74) Representative: Stüven, Ralf

(57) **Abstract**

The invention relates to an antibody for use in a therapy involving the cytotoxic elimination of a target cell by engaging the target cell with an immune effector cell, the antibody comprising a first binding domain binding to an epitope on a surface protein present on the surface of the target cell and a second binding domain binding to an epitope on a surface protein present on the surface of the immune effector cell, wherein the surface protein on the surface of the target cell is also present on the surface of the immune effector cell.

## Description

The invention relates to an antibody for use in a therapy involving the cytotoxic elimination of a target cell by engaging the target cell with an immune effector cell.

Immune cell engagers (ICEs) like, for example, bispecific antibodies are promising tools for use in immunotherapy (see, e.g., Fucà G, Spagnoletti A, Ambrosini M, de Braud F, Di Nicola M, 2021, Immune cell engagers in solid tumors: promises and challenges of the next generation immunotherapy, ESMO Open 6, 100046, doi: /10.1016/j.esmoop.2020.100046). Bispecific antibodies (BsAbs) are antibodies or antibody constructs having two different paratopes, thus being able to bind to two different epitopes at the same time. BsAbs are increasingly used in medical therapy, for example, cancer therapy (see, for example, Suurs FV, Lub-de Hooge MN, de Vries EGE., de Groot DJA., 2019, A review of bispecific antibodies and antibody constructs in oncology and clinical challenges, Pharmacology & Therapeutics 201, 103-119, DOI: 10.1016/j.pharmthera.2019.04.006; Bhatta P, Whale KD, Sawtell AK, Thompson CL, Rapecki SE, Cook DA, Twomey BM, Mennecozzi M, Starkie LE, Barry EMC, Peters SJ, Kamal AM, Finney HM, 2021, Bispecific antibody target pair discovery by high-throughput phenotypic screening using in vitro combinatorial Fab libraries, mAbs 13:1, 1859049, DOI: 10.1080/19420862.2020.1859049; Velasquez MP, Bonifant CL, Gottschalk S., 2018, Redirecting T cells to hematological malignancies with bispecific antibodies, Blood 131(1):30-38, DOI: 10.1182/blood-2017-06-741058; Gera N, 2022, The evolution of bispecific antibodies, Expert Opinion on Biological Therapy, 22:8, 945-949, DOI: 10.1080/14712598.2022.2040987; Zhu Y, Shen R, Hao R, Wang S, Ho M., Highlights of Antibody Engineering and Therapeutics 2019 in San Diego, USA: Bispecific Antibody Design and Clinical Applications, 2020, Antib Ther. 3(2):146-154, DOI: 10.1093/abt/tbaa012; Sedykh SE, Prinz VV, Buneva VN, Nevinsky GA, 2018, Bispecific antibodies: design, therapy, perspectives, Drug Des Devel Ther. 12:195-208, DOI: 10.2147/DDDT.S151282; Wu Y, Yi M, Zhu S, Wang H, Wu K, 2021, Recent advances and challenges of bispecific antibodies in solid tumors, Experimental Hematology & Oncology, 10:56, DOI: 10.1186/s40164-021-00250-1; Ma J, Mo Y, Tang M, Shen J, Qi Y, Zhao W, Huang Y, Xu Y and Qian C, 2021, Bispecific Antibodies: From Research to Clinical Application, Front. Immunol. 12:626616, DOI: 10.3389/fimmu.2021.626616; Wang S, Chen K, Lei Q, Ma P, Yuan AQ, Zhao Y, Jiang Y, Fang H, Xing S, Fang Y, Jiang N, Miao H, Zhang M, Sun S, Yu Z, Tao W, Zhu Q, Nie Y, Li N, 2021, The state of the art of bispecific antibodies for treating human malignancies, EMBO Mol Med (2021) 13: e14291, DOI: 10.15252/emmm.202114291). In particular, BsAbs, which can bind a target cell (e.g., tumor cell) and an effector cell (e.g., T cell) at the same time, are now a well-tested and promising treatment option for many diseases. They enable the activation ("engagement") of the effector cells and in consequence the cytotoxic elimination of the target cells by the effector cells. Only a few bispecific antibodies, including, for example, Blinatumomab, have been approved for clinical use to date (see, e.g., Wang S, Chen K, Lei Q, Ma P, Yuan AQ, Zhao Y, Jiang Y, Fang H, Xing S, Fang Y, Jiang N, Miao H, Zhang M, Sun S, Yu Z, Tao W, Zhu Q, Nie Y, Li N, 2021, The state of the art of bispecific antibodies for treating human malignancies, EMBO Mol Med (2021) 13: e14291, DOI: 10.15252/emmm.202114291; Ma J, Mo Y, Tang M, Shen J, Qi Y, Zhao W, Huang Y, Xu Y and Qian C, 2021, Bispecific Antibodies: From Research to Clinical Application, Front. Immunol. 12:626616, DOI: 10.3389/fimmu.2021.626616).

There is still a need for novel antibodies for use in therapy, in particular immunotherapy, for example cancer immunotherapy. It is an object of the invention to provide such an antibody, in particular an antibody for use as an immune cell engager with a wider application range than in the prior art.

The object is solved by an antibody for use in a therapy involving the cytotoxic elimination of a target cell by engaging the target cell with an immune effector cell, the antibody comprising a first binding domain binding to an epitope on a surface protein present on the surface of the target cell and a second binding domain binding to an epitope on a surface protein present on the surface of the immune effector cell, wherein the surface protein on the surface of the target cell is also present on the surface of the immune effector cell.

So far, the basic prerequisite for the functioning of the principle of target cell and effector cell engaging antibodies, e.g. bispecific antibodies, has been that the target antigens are only expressed on the target cell but not at all or at substantially lower levels on the effector cells, since the fratricide to be expected would impede the effectiveness of the principle. This concern is even more pronounced when the target antigen is expressed at high levels on the effector cells. It has therefore been a generally accepted view so far that an antigen such as, for example, CD45, which is highly and ubiquitously expressed on immune effector cells, is not a suitable target antigen for bispecific antibodies. The present inventors have found, however, that, contrary to this assumption, it is possible to advantageously use, for example, bispecific antibodies in such a context.

The antibody of the invention for use in a therapy involving the cytotoxic elimination of a target cell by engaging the target cell with an immune effector cell is an artificial construct configured to comprise at least two binding domains with which two epitopes on two spatially separated molecules can be bound, e.g., two epitopes on two surface proteins that are present on the surfaces of two cells. The antibody is thus designed with regard to its physical structure, and in particular as regards the spatial distance between and/or the orientation of its two binding domains, in a manner enabling the antibody to engage a target cell with an immune effector cell by simultaneously binding on the one hand to an epitope on the surface of the target cell and on the other hand to an epitope on the surface of the immune effector cell. The term "target cell and effector cell engaging antibody" may also be used herein in relation to such an artificial antibody according to the invention. The term "simultaneously binding" does not exclude that the antibody can bind to both epitopes one after another, but means that both epitopes are bound by the antibody over a common period of time. The antibody can be an antibody composed of a single polypeptide or of two or more polypeptides being covalently bound together, e.g., via disulfide bonds, or forming a peptide complex (dimer or oligomer) composed of separate polypeptide chains (subunits) held together by noncovalent forces to form a quaternary structure. The antibody of the invention can be a multi-specific, e.g., trispecific, or a bispecific antibody. A bispecific antibody is preferred. The two epitopes can be identical (though on two separate molecules of the same kind) or different from each other.

The term "immunological synapse" is used herein in relation to a cell-cell junction with a synaptic cleft stabilized by an adhesion molecule, e.g., bispecific antibody, enabling cell-cell communication between an immune cell and a target cell. The term "cytolytic synapse" relates to an immunological synapse formed for cytolytically eliminating a target cell by or with the aid of an immune cell.

The term "multi-specific antibody" relates to an artificial antibody that can simultaneously bind two or more separate and unique epitopes, the epitopes being on separate antigens or on the same antigen. As used herein, the term also encompasses an artificial antibody that can simultaneously bind to the same epitope or different epitopes on two or more separate but identical antigens, e.g., two or more separate surface molecules of the same kind (e.g., CD3) present on, e.g., two separate cells. The term also encompasses bispecific antibodies.

The term "bispecific antibody", abbreviated BsAb, relates to an artificial antibody that can simultaneously bind two separate and unique epitopes, the epitopes being on two separate antigens or on the same antigen (see, for example, the references above). As used herein, the term also encompasses an artificial antibody that can simultaneously bind to the same epitope or different epitopes on two separate but identical antigens, e.g., two separate surface molecules of the same kind (e.g., CD3) present on two separate cells. As used herein, the term "bispecific antibody" should not be construed as meaning that further specificities, for example a third specificity, are excluded. The term thus encompasses also trispecific or multi-specific antibodies. Bispecific antibodies are designed to have two binding domains (paratopes) with preferably different defined specificities. They can be produced, for example, by various chemical, biochemical or biotechnological means, and exist in many different formats (see, e.g., Brinkmann U, Kontermann RE, 2017, The making of bispecific antibodies, MAbs. 9(2):182-212, DOI: 10.1080/19420862.2016.1268307; Voigt J, Meyer C, Bordusa F, 2022, Synthesis of Multiple Bispecific Antibody Formats with Only One Single Enzyme Based on Enhanced Trypsiligase, Int. J. Mol. Sci. 23, 3144, DOI: 10.3390/ijms23063144). BsAbs may or may not include a functional antibody Fc-region.

The term "epitope" (also called "antigenic determinant") relates to a portion of a substance, e.g., a molecule, molecular structure or particulate matter, called antigen, to which a specific antibody binds with its binding domain. The term "antigen" relates to a substance, e.g., a molecule, molecular structure or particulate matter, that comprises at least one epitope and that is capable of stimulating an immune response, in particular is recognized by an antibody. The terms "antigen" and "epitope" may be used synonymously here, in particular in a case where the two epitopes are present on different molecules. For example, saying that the bispecific antibody binds an epitope on CD45 is equivalent to saying that the BsAb binds to the CD45 antigen, if not clearly stated otherwise.

The term "therapy" relates to a medical treatment of a disease or disorder. The term "immunotherapy" relates to a medical treatment of a disease or disorder by activating or suppressing the immune system of the subject to be treated. An immunotherapy can, for example, be a cell-based therapy, i.e., a therapy involving the activation or suppression of immune effector cells. The term "cancer immunotherapy" relates to an immunotherapy, in particular an antibody and/or cell-based immunotherapy, for treating cancer, for example a hematopoietic cancer.

The term "involving the cytotoxic elimination of a target cell by engaging the target cell with an immune effector cell" in relation to a therapy relates to a therapy in which immune effector cells are brought in intimate contact with a target cell, e.g., using artificial molecules like bispecific antibodies, thus forming a cytolytic synapse leading to cell death of the target cell.

The terms "immune effector cell", IEC, "effector immune cell" or "effector cell" relate to cells of the immune system of the human body, i.e., cells that are capable of modulating, effecting or enhancing an immune response in response to stimulation. Examples of immune effector cells include natural killer (NK) cells, cytotoxic T lymphocytes (cytotoxic T cells, CD8+ cells), B lymphocytes (B cells), helper T cells (T helper cells, CD4+ cells), macrophages and dendritic cells. The term also encompasses genetically modified immune effector cells.

The term "cytotoxic elimination" relates to the killing of cells, e.g., cancer cells, e.g. by immune effector cells.

The term "target cell" relates to a living cell, e.g., a cancer cell, being a target for cytotoxic elimination. The term encompasses immune effector cells being targeted, e.g., cancerous T cells as in the case of acute leukemia, for example.

The term "fratricide" relates to the cytotoxic elimination of effector cells by effector cells of the same type, e.g., of NK cells by other NK cells or of T cells by other T cells.

The term "T cell engager" (TCE) relates to a protein simultaneously binding a target antigen on a target cell, e.g., a tumor cell, and an antigen on a T cell to form an artificial immune synapse that is independent of the T cell receptor (TCR) (see, for example, Vafa O, Trinklein ND, 2020, Perspective: Designing T-Cell Engagers With Better Therapeutic Windows, Front. Oncol. 10: 446, DOI: 10.3389/fonc.2020.00446). A bispecific antibody can, for example, be a T cell engager (bispecific T-cell engager, BiTE^{®}) and circumvent HLA restriction. In case of a bispecific T cell engager, the term bispecific T-cell engager, abbreviated, "BTCE" or "bTCE", may, for example, be used. Similarly, the term "NK cell engager" (NKCE) relates to a protein simultaneously binding a target antigen on a target cell, e.g., a tumor cell, and an antigen on an NK cell to form an artificial immune synapse. Again, a bispecific antibody can function as an NK cell engager (BiKE or BNKCE) (see, e.g., Demaria O, Gauthier L, Debroas G, Vivier E, 2021, Natural killer cell engagers in cancer immunotherapy: Next generation of immunooncology treatments, Eur. J. Immunol. 51: 1934-1942, DOI:10.1002/eji.202048953; Sivori S, Meazza R, Quintarelli C, Carlomagno S, Della Chiesa M, Falco M, Moretta L, Locatelli F, Pende D, 2019, NK Cell-Based Immunotherapy for Hematological Malignancies, J Clin Med. 8(10):1702, DOI: 10.3390/jcm8101702; Felices M, Lenvik TR, Davis ZB, Miller JS, Vallera DA, 2016, Generation of BiKEs and TriKEs to Improve NK Cell-Mediated Targeting of Tumor Cells, Methods Mol Biol. 1441:333-46, DOI: 10.1007/978-1-4939-3684-7_28).

The terms "hematopoietic cancer" or "hematologic cancer" relate to a cancer of the hematopoietic system, in particular the blood cells and their progenitors. Blood cells comprise, for example, erythrocytes, leukocytes, plasma cells and thrombocytes. Examples of hematologic cancer are leukemia, lymphoma, or multiple myeloma.

The terms "CD45" (cluster of differentiation 45) or "CD45 antigen" have their common meanings and relate to a transmembrane protein that is a member of the protein tyrosine phosphatase (PTP) family. The protein is also known as PTPRC (Protein tyrosine phosphatase, receptor type, C), and is encoded by the *PTPRC* gene. CD45 is present in several isoforms on all differentiated nucleated hematopoietic cells. The term also encompasses all CD45 isoforms.

The terms "CD3" (cluster of differentiation 3) or "CD3 antigen", also "CD3 receptor", have their common meanings and relate to a protein complex comprising four different transmembrane proteins, in mammals a gamma chain, a delta chain and two epsilon chains. The protein complex associates with the T-cell receptor (TCR). CD3 is, almost exclusively, present on mature T cells.

The terms "CD16" (cluster of differentiation 16) or "CD16 antigen", also known as FcyRIII, is a membrane receptor protein. The term encompasses the two variants existing in humans, CD16a (FcyRIIIa) and CD16b (FcyRIIIb). CD16 is found on the surface of natural killer cells, neutrophils, monocytes, macrophages, and certain T cells.

Terms in the format of "first surface protein x second surface protein", e.g. "CD3xCD16", "CD3xCD45" or "CD3xCD3", relate to antibodies, e.g. bispecific antibodies, having a first paratope binding to an epitope on the first surface protein (e.g. CD3) and a second paratope binding to an epitope on a second surface protein (e.g. CD 16). A "CD3xCD16" antibody is thus an antibody, e.g., bispecific antibody, binding, with a first binding domain, to an epitope on CD3 and, with a second binding domain, to an epitope on CD16. As another example, a "CD3xCD3" antibody is an antibody, for example, bispecific antibody, binding, with a first binding domain, to an epitope on a CD3 protein and, with a second binding domain, to an epitope on another CD3 protein, the epitope on the other CD3 protein being the same or different from the first epitope.

The term "surface protein" relates to a membrane protein, i.e., a protein being part of or interacting with a biological membrane, in particular to a membrane protein having at least an extracellular portion. Surface proteins may be designated with their common abbreviations known to the skilled person, e.g. "CD3", "CD16", "CD38" or "CD45". A term like "CD#", "CD" standing for "Cluster of Differentiation" and "#" representing a number or a combination of a number and a letter (e.g., 3 or 45 or 1a), for example "CD45", is to be understood as also encompassing all isoforms, in case of CD45, for example, the isoforms CD45RA and CD45RO. A term like, for example, "CD45-positive leukemia cells" thus also includes CD45RA-positive leukemia cells. Further, use of a term like "CD#", e.g., CD43, encompasses proteins being, for example, post-translationally modified, e.g., glycosylated or sialylated.

The term "present on the surfaces of a cell" in relation to a surface protein relates to the presence of the protein or a portion thereof on or the accessibility of the protein or a portion thereof from the extracellular side of a biological membrane.

The term according to which "a surface protein on the surface of the target cell is also present on the surface of the immune effector cell" is not to be construed as meaning that one and the same protein molecule is present on both the target and the immune effector cell. Rather, the term relates to the fact that the same type of surface protein, each being composed, for example, of (a) polypeptide(s) having essentially the same amino acid sequence, secondary, tertiary and quaternary structure, is present on both the target and the immune effector cell. For example, a first CD45 molecule may be present on the target cell and a second CD45 molecule on the immune effector cell. Likewise, an expression according to which "the surface proteins and the epitopes on the target cell and on the immune effector cell are identical" is not to be construed as meaning that one and the same protein molecule or epitope is present on the target cell and on the immune effector cell, but that the same kind of molecule is present on the target cell and the immune effector cell, e.g., a protein molecule belonging to the class of CD45 molecules.

The term "expression level" in relation to a surface protein relates to the level of expression of the protein or a portion thereof or the accessibility of the protein or a portion thereof from the extracellular side of a biological membrane as determined, e.g., by flow cytometry, e.g., according to standards set by the HCDM (human cell differentiation molecules organization, see, e.g. Kužílková D, Puñet-Ortiz J, Aui PM, et al., 2022, Standardization of Workflow and Flow Cytometry Panels for Quantitative Expression Profiling of Surface Antigens on Blood Leukocyte Subsets: An HCDM CDMaps Initiative, Front Immunol. 13:1-15, doi: 10.3389/fimmu.2022.827898; Kalina T, Fišer K, Pérez-Andrés M, Kuzílková D, Cuenca M, Bartol SJWW, Blanco E, Engel P, van Zelm MC, 2019, CD Maps-Dynamic Profiling of CD1-CD100 Surface Expression on Human Leukocyte and Lymphocyte Subsets., Front Immunol 10:2434. doi:10.3389/fimmu.2019.02434). An expression, according to which a surface protein is present on the surface of a first cell at the same or at a higher level than on the surface of a second cell thus means that the surface protein is expressed in the first cell at the same or a higher level than in the second cell, or is at least extracellularly accessible on the first cell at the same or a higher level than on the second cell, e.g., for the binding of an antibody to it. In particular, the expression relates to the number of molecules or molecule portions of a surface protein that are extracellularly accessible for a particular binding molecule, e.g. an antibody.

The term "pharmaceutically acceptable" means any substantially non-toxic material for mammals, especially humans, which does not substantially affect the effectiveness of the biological activity of the active ingredient. A "non-toxic" material is a material that is not toxic in the amount or concentration used per se or in the context of the composition administered. Such materials may include pharmaceutically acceptable concentrations of salts, buffers, preservatives, or the like. Non-limiting examples of pharmaceutically acceptable carriers include magnesium carbonate, magnesium stearate, talc, sugar, lactose, ethanol, glycerol, water, buffer solutions. The pharmaceutical composition may also comprise adjuvants and/or diluents.

The terms "complement activity activating or directing capability" or "capability of activating or directing complement activity" in relation to an antibody of the invention refers to the property of the antibody to be able to activate or direct the complement system of the immune system, based on a structural element or structural elements of the antibody, for example, a C1q binding Fc domain, leading to activation, amplification or modification of the complement system (see, e.g., Wang G, de Jong RN, van den Bremer ETJ, Beurskens FJ, Labrijn AF, Ugurlar D, Gros P, Schuurman J, Parren PWHI, Heck AJR, 2016, Molecular Basis of Assembly and Activation of Complement Component C1 in Complex with Immunoglobulin G1 and Antigen, Molecular Cell 63, 135-145, doi:10.1016/j.molcel.2016.05.016; Peschke B, Keller CW, Weber P, Quast I, Lünemann JD, 2017, Fc-Galactosylation of Human Immunoglobulin Gamma Isotypes Improves C1q Binding and Enhances Complement-Dependent Cytotoxicity, Frontiers in Immunology 8, doi: 10.3389/fimmu.2017.00646; Lee CH, Romain G, Yan W, Watanabe M, Charab W, Todorova B, Lee J, Triplett K, Donkor M, Lungu OI, Lux A, Marshall N, Lindorfer MA, Goff OR, Balbino B, Kang TH, Tanno H, Delidakis G, Alford C, Taylor RP, Nimmerj ahn F, Varadaraj an N, Bruhns P, Zhang YJ, Georgiou G, 2017, IgG Fc domains that bind C1q but not effector Fcγ receptors delineate the importance of complement-mediated effector functions, Nat Immunol 18: 889-898, doi: 10.1038/ni.3770; Sörman A, Zhang L, Ding Z, Heyman B, 2014, How antibodies use complement to regulate antibody responses, Molecular Immunology 61, 79-88, doi:10.1016/j.molimm.2014.06.010; Goldberg BS, Ackerman ME, 2020. Antibody-mediated complement activation in pathology and protection, Immunol Cell Biol 98:305-317, doi:10.1111/imcb.12324).

The invention provides an antibody for use in a therapy involving the cytotoxic elimination of a target cell by engaging the target cell with an immune effector cell. The target cell and effector cell engaging antibody is configured to being able to bind to an epitope on a surface protein present on the surface of a target cell and simultaneously to an epitope on a surface protein present on the surface of an immune effector cell in order to enable the formation of a synapse between the target cell and the immune effector cell that would finally lead to the cell death of the target cell. The antibody thus comprises at least two paratopes binding to epitopes on two separate surface protein molecules. The antibody of the invention can efficiently be used in a therapy involving the cytotoxic elimination of a target cell by engaging the target cell with an immune effector cell, although the surface protein on the surface of the target cell is also present on the surface of the immune effector cell. The surface proteins and the epitopes on the target cell and immune effector cell may be identical, e.g., CD3 molecules on the target and the immune effector cell, or different from each other, the latter being preferred.

It is preferred that the surface protein that is present on the surface of the target cell and also on the surface of the immune effector cell is present on the surface of the immune effector cell at the same or at a higher level than on the surface of the target cell. This is preferred both in case the surface proteins and/or the epitopes on the target cell and the immune effector cell are identical or different from each other.

In a preferred embodiment of the antibody for use in a therapy involving the cytotoxic elimination of a target cell by engaging the target cell with an immune effector cell, the antibody is configured to bind to a first epitope on the target cell and simultaneously to a second epitope on the immune effector cell, wherein the first epitope is present on both the target cell and the immune effector cell, whereas the second epitope is present on the immune effector cell, but preferably absent on the target cell. This is particularly preferred in case the target cell is not an immune effector cell. First and second epitope are preferably different from each other, and preferably are on different antigens, i.e., different surface proteins. In case the first and second epitope are different from each other, it is particularly preferred that the second epitope is only present on the immune effector cell, but not or at least at a lower level on the target cell. Preferably, the second epitope is present on the target cell at a level of not more than 60%, preferably not more than 50%, 40%, 30%, 20%, 15%, 10%, 5%, 2% or 1% of the level at which the epitope is present on the immune effector cell. Most preferred, the second epitope is not present on the target cell. The term "different surface proteins" also encompasses surface proteins having the same amino acid backbone, but being differently glycosylated or sialylated, such that an antibody binding to a first surface protein would not bind to a second surface protein having the same amino acid sequence and corresponding tertiary or quaternary structure, but being differently glycosylated or sialylated. A sialylated CD43 (CD43s), for example, is, in this context, considered different from a non-sialylated CD43, to which an antibody binding to CD43s would not bind (Bartels L, de Jong G, Gillissen MA, Yasuda E, Kattler V, Bru C, Fatmawati C, van Hal-van Veen SE, Cercel MG, Moiset G, Bakker AO, van Helden PM, Villaudy J, Hazenberg MD, Spits H, Wagner K, 2019, A Chemo-enzymatically Linked Bispecific Antibody Retargets T Cells to a Sialylated Epitope on CD43 in Acute Myeloid Leukemia. Cancer Res 79 (13): 3372-3382, doi:10.1158/0008-5472.CAN-18-0189). As another example, a variant of a CD43 protein having a unique epitope not present on normal CD43 proteins, is considered a different surface protein.

In an embodiment of the antibody for use in a therapy involving the cytotoxic elimination of a target cell by engaging the target cell with an immune effector cell, wherein the target cell is also an immune effector cell, the second epitope can also be present on the target cell (target immune effector cell) at the same or a higher level than on the immune effector cell. Contrary to what was previously assumed, the expected fratricide does not lead to a significant impairment of the therapeutic effect. The invention thus provides a possibility to target cells, using target cell and effector cell engaging antibodies like bispecific antibodies, that previously could not be targeted or at least not be targeted using, for example, bispecific antibodies binding to an epitope that is present on the target cell, but would also be present, in particular present at the same or high levels, on the immune effector cells. The invention can advantageously be used, for example, in the therapeutic elimination of immune cells, the therapy of blood-cell derived malignancies or immune-mediated diseases, or in the context of conditioning prior to stem cell or organ transplantation.

In preferred embodiments of the antibody of the invention, the epitope present on both the target and immune effector cell is an epitope selected from the group consisting of CD2, CD3, CD5, CD6, CD7, CD11a, CD16, CD17, CD18, CD28, CD43, CD44, CD45, CD46, CD48, CD50, CD53, CD99, CD122, CD130, CD150, CD159a, CD159c, CD183, CD192, CD210, CD226, CD247, CD305, CD335, and CD337.

In a particularly preferred embodiment of the antibody for use in a therapy involving the cytotoxic elimination of a target cell by engaging the target cell with an immune effector cell, the first epitope, i.e., the epitope being present on both the target and the immune effector cell, is an epitope on CD45. In this embodiment, the antibody of the invention is particularly useful for targeting hematopoietic cells, e.g., blood cancer cells like CD45-positive leukemia cells. CD45 is ubiquitously found on nucleated hematopoietic cells and highly expressed on immune effector cells, but not on any non-hematopoietic cells.

In a further particularly preferred embodiment of the antibody for use in a therapy involving the cytotoxic elimination of a target cell by engaging the target cell with an immune effector cell, of the invention, the second epitope is an epitope on CD3 or CD16. Examples of suitable antibodies of the invention targeting CD45 on the one hand, and CD3 or CD16 on the other hand, in this case bispecific antibodies, are antibodies whose sequences are given in SEQ IDs NO: 1-4, SEQ ID NO: 2 representing the amino acid of a CD45xCD3 BiTE encoded by the nucleotide sequence of SEQ ID NO: 1, SEQ ID NO: 4 representing the amino acid of a CD45xCD16 BiKE encoded by the nucleotide sequence of SEQ ID NO: 3.

In a further preferred embodiment of the invention, the antibody for use in a therapy involving the cytotoxic elimination of a target cell by engaging the target cell with an immune effector cell is not a bispecific CD38xCD3 antibody.

In a preferred embodiment of the antibody for use in a therapy involving the cytotoxic elimination of a target cell by engaging the target cell with an immune effector cell, the immune effector cell is a T cell, preferably a cytotoxic T cell, or a natural killer cell. In this embodiment, the antibody is configured to bind, with its second binding domain, to a second epitope on a T cell or a natural killer cell as an immune effector cell. In case the immune effector cell is a T cell, it is particularly preferred that the second epitope is an epitope on CD3. In case the immune effector cell is a natural killer cell, it is particularly preferred that the second epitope is an epitope on CD16. In particularly preferred embodiments, the first epitope is CD45.

In a preferred embodiment of the antibody of the invention, wherein the surface proteins and/or the epitopes on the target cell and on the immune effector cell are identical, the surface protein on the target cell and the surface protein on the immune effector cell are both CD3.

In a preferred embodiment of the antibody for use in a therapy involving the cytotoxic elimination of a target cell by engaging the target cell with an immune effector cell, the antibody is configured to engage a cancer cell, preferably a malignant hematopoietic cell, as the target cell with the immune effector cell.

The antibody can have any suitable format. The antibody can be a multispecific, trispecific or bispecific antibody. In a preferred embodiment the antibody is a bispecific antibody, preferably a bispecific antibody with two paratopes directed against different epitopes. The skilled person is familiar with, for example, bispecific antibodies and methods for their synthesis (see, for example, Voigt J, Meyer C, Bordusa F, 2022, Synthesis of Multiple Bispecific Antibody Formats with Only One Single Enzyme Based on Enhanced Trypsiligase, Int. J. Mol. Sci. 23, 3144, DOI: 10.3390/ijms23063144).

The invention also relates to a pharmaceutical composition or dosage form for use in a therapy involving the cytotoxic elimination of a target cell by engaging the target cell with an immune effector cell, the pharmaceutical composition comprising an antibody for use in a therapy involving the cytotoxic elimination of a target cell by engaging the target cell with an immune effector cell as described above, and a pharmaceutically acceptable carrier. The composition may, for example, be soluble or in liquid form for parenteral, e.g., intravenous, administration.

In a further aspect, the invention relates to a therapeutic method involving the cytotoxic elimination of a target cell by engaging the target cell with an immune effector cell, wherein an antibody is administered to a subject in need of such a therapy in a therapeutically effective amount, the antibody comprising a first binding domain binding to an epitope on a surface protein present on the surface of the target cell and a second binding domain binding to an epitope on a surface protein present on the surface of the immune effector cell, wherein the surface protein on the surface of the target cell is also present on the surface of the immune effector cell.

In a preferred embodiment of the therapeutic method of the invention involving the cytotoxic elimination of a target cell by engaging the target cell with an immune effector cell, the antibody administered to a subject in need of such a therapy in a therapeutically effective amount comprises a first binding domain binding to a first epitope and a second binding domain binding to a second epitope, the first and second epitope being different from each other, wherein the antibody is configured to bind, with its first binding domain, to the first epitope, the first epitope being present on a first surface protein present on the surface of the target cell and, preferably at the same or a higher level, on the surface of the immune effector cell, and with its second binding domain to the second epitope, the second epitope being present on a surface protein present on the surface of the immune effector cell, but preferably absent on the target cell.

In a preferred embodiment, the antibody, for example bispecific antibody, is administered to the patient intravenously. In another embodiment, the bispecific antibody is administered to the patient by another parenteral route.

The antibody, e.g. bispecific antibody, may be administered as a single agent or in combination with one or more other agents such as irradiation, cytotoxic drugs or other anti-cancer agents. In this regard, the antibody may be administered simultaneously, before or after the other agent or agents.

In a preferred embodiment of the method of the invention, the first epitope, i.e., the epitope being present on both the target and the immune effector cell, is an epitope on CD45. In this embodiment, the method of the invention is particularly useful for targeting hematopoietic cells, e.g., blood cancer cells like CD45-positive leukemia cells. In a further preferred embodiment of the method of the invention the second epitope is an epitope on CD3 or C16.

In a further preferred embodiment of the method of the invention, the immune effector cell is a T cell, e.g., cytotoxic T cell, or a natural killer cell. In this embodiment, the antibody is configured to bind, with its second binding domain, to a second epitope on a T cell or a natural killer cell as an immune effector cell. Preferably, first and second epitope are different from each other. In case the immune effector cell is a T cell, it is particularly preferred that the second epitope is an epitope on CD3. In case the immune effector cell is a natural killer cell, it is particularly preferred that the second epitope is an epitope on CD16. In particularly preferred embodiments, the first epitope is CD45 in case.

In a preferred embodiment of the method of the invention, the antibody, for example bispecific antibody, is configured to engage a cancer cell, preferably a malignant hematopoietic cell, as the target cell with the immune effector cell.

In a particular preferred embodiment, the antibody, e.g., bispecific antibody, is configured to being also capable of activating or directing complement activity to the target cell via, e.g., Fc domains or other complement binding domains.

In order to avoid unnecessary redundancy, and for clarity reasons, it is to be noted that the invention covers any therapeutic method involving the cytotoxic elimination of a target cell by engaging the target cell with an immune effector cell, wherein any of the above-mentioned embodiments of the antibody described above is administered to a subject in need of such a therapy in a therapeutically effective amount.

The target cell can also be an immune effector cell.

The invention is explained in more detail below with reference to the accompanying figures and exemplary embodiments for illustrative purposes only.

### BRIEF DESCRIPTION OF THE DRAWINGS

FIG. 1 Schematic drawing showing the basic concept of the invention.
FIG. 2 Results of a luciferase assay of the killing of Ramos-Luc2 cells with CD45 BsAbs in the presence of KHYG-1 CD16+ natural killer cells after 4 hours incubation.
FIG. 3 Results of a luciferase assay of the killing of Ramos-Luc2 cells with CD45 BsAbs in the presence of primary T cells BC#12 after 22 hours incubation.

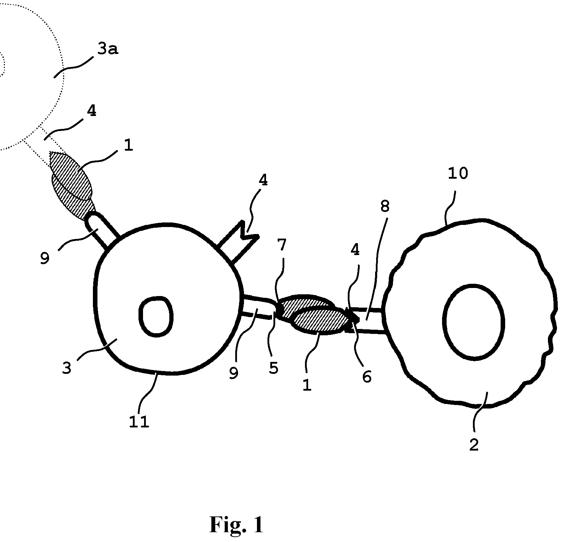

Figure 1 schematically illustrates the basic concept of a preferred embodiment of the invention. A bispecific antibody 1 binds, with its first binding domain (paratope) 6, to a first epitope 4 on a first surface protein 8, e.g., CD45, on the surface 10 of a target cell 2, e.g., a malignant lymphocyte, and, with its second binding domain (paratope) 7, to a second epitope 5 on a second surface protein 9, e.g., CD3 or CD16, on the surface 11 of an immune effector cell 3, for example a T cell or NK cell. The first surface protein 8 and the second surface protein 9 are different from each other, as are the first epitope 4 and second epitope 5. In this manner, the immune effector cell 3, for example a T cell or natural killer cell (NK cell), and the target cell 2 are engaged with each other via the bispecific antibody, BsAb, 1. Both the target cell 2 and the immune effector cell 3 express and carry on their surfaces 10, 11, the same first surface protein 8 with the first epitope 4 that can be bound by the first binding domain 6 of the BsAb 1. Here, only the immune effector cell 3 carries the second surface protein 9 with a second epitope 5 on its surface 11. In the upper left corner of figure 1, a fratricide situation is schematically depicted. Another immune effector cell 3a that also carries the first surface protein 4, e.g., CD45, on its surface, is bound via the BsAb 1 to the immune effector cell 3, which can lead to the killing of the other immune effector cell 3a. Although such a fratricide occurs when the bispecific antibody according to the invention is used, it has been found that this does not decisively impair the success of a therapy involving the administration of the bispecific antibody.

### Examples

### 1. Use of a bispecific NK cell engager

A bispecific NK cell engager (BiKE) was developed, whose binding domains are directed against CD45 and CD16 (CD45xCD16 BiKE, protein in SEQ ID NO: 4, coding sequence in SEQ ID NO: 3). An scFv from a previously described monoclonal antibody (Lin Y, Pagel JM, Axworthy D, Pantelias A, Hedin N, Press OW, A Genetically Engineered Anti-CD45 Single-Chain Antibody-Streptavidin Fusion Protein for Pretargeted Radioimmunotherapy of Hematologic Malignancies, 2006, Cancer Res 66 (7): 3884-3892, DOI:10.1158/0008-5472.CAN-05-3443) was used in the BiKE to bind the CD45. This scFv is encoded by a new, codon-optimized cDNA designed by the applicants. CD16 is bound via a heavy-chain antibody ("Nanobody^{®}") directed against this molecule. The CD16 nanobody^{®} used is known from the prior art (see, for example, Behar G, Sibéril S, Groulet A, Chames P, Pugnière M, Boix C, Sautès-Fridman C, Teillaud JL, Baty D., Isolation and characterization of anti-FcgammaRIII (CD16) llama single-domain antibodies that activate natural killer cells, 2008, Protein Eng Des Sel. 21(1): 1-10, DOI: 10.1093/protein/gzm064;US 2018/0022826 A1).

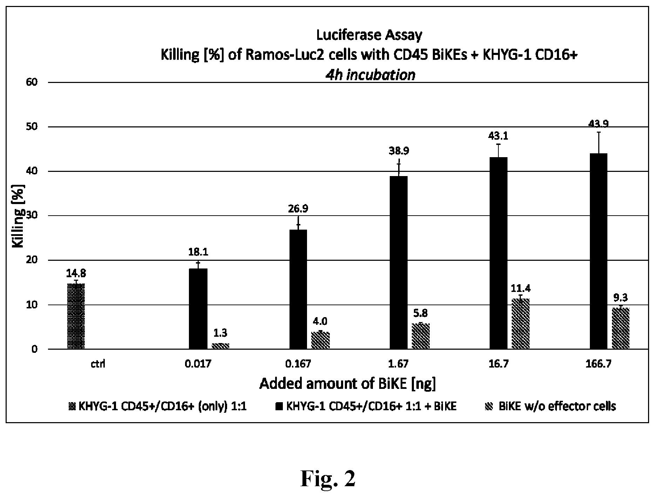

As shown in Fig. 2, the CD45xCD16 BiKE mediated an efficient lysis (ADCC) of CD45-positive leukemia cells (Ramos-Luc2 cells) in the presence of CD16-positive NK cells (line KHYG-1) within only four hours, even with a low effector cell : target cell ratio (1:1). As expected, the effect was concentration-dependent and was achieved with both CD45-positive (Fig. 2) and CD45-negative NK cells (not shown).

### 2. Use of a bispecific T cell engager

A bispecific (CD45xCD3) T-cell engager (Bhatta P, Whale KD, Sawtell AK, Thompson CL, Rapecki SE, Cook DA, Twomey BM, Mennecozzi M, Starkie LE, Barry EMC, Peters SJ, Kamal AM, Finney HM, 2021, Bispecific antibody target pair discovery by high-throughput phenotypic screening using in vitro combinatorial Fab libraries, mAbs 13:1, 1859049, DOI: 10.1080/19420862.2020.1859049) was cloned. For this purpose, the CD45-directed scFv described above was coupled with an anti-CD3-directed scFv. The latter was derived from the known bispecific T-cell engager blinatumomab, which is already routinely used clinically in B-cell leukemias. As above, the DNA sequence of the CD3-scFv has been codon-optimized and newly synthesized for optimal expression. The amino acid sequence is given in SEQ ID NO: 2, the corresponding coding sequence in SEQ ID NO: 1.

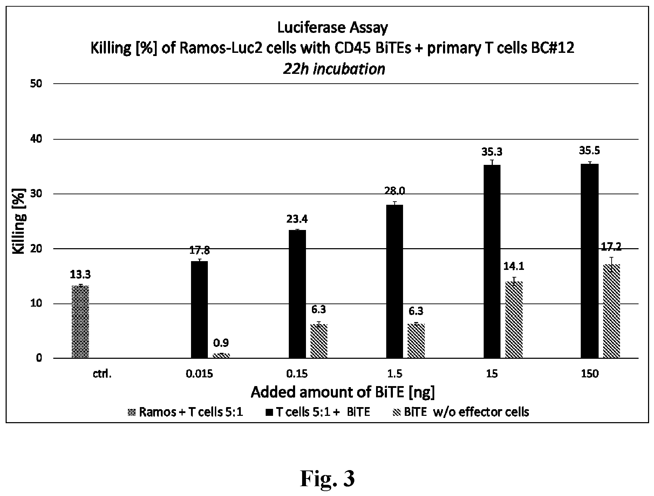

After 22 hours of incubation in the presence of primary T lymphocytes from healthy donors, the CD45xCD3 BsAb also mediated a clearly concentration-dependent lysis of the CD45-positive Ramos leukemia cells (Fig. 3).

### Sequences:

SEQ ID NO: 1 (CD45xCD3 T-cell engager, DNA): 
atgggatggagctgtatcatcctcttcttggtagcaacagctacaggcgcgcactccatggaagtgaagctgctggaatctggcggcgga
ctggttcaacctggcggatctctgaagctgagctgtgccgccagcggcttcgacttcagcagatactggatgagctgggtccgacaggc
ccctggcaaaggactggaatggatcggcgagatcaaccccaccagcagcaccatcaacttcacccctagcctgaaggacaaggtgttc
atcagccgggacaacgccaagaacaccctgtacctgcagatgagcaaagtgcggagcgaggacacagccctgtactactgtgccaga
ggcaactactacagatacggcgacgctatggactactggggccagggaacaagcgtgaccgtgtctaagatttctggcggaggcggat
caggcggcggaggaagcggtggcggaggatcaggtggtggtggatcaggcggaggtggcagctctgatatcgtgctgacacagtct
ccagccagcctggccgtttctctgggacagagagccaccatcagctgcagagccagcaagagcgtgtccacaagcggctacagctac
ctgcactggtatcagcagaagcccggccagcctcctaagctgctgatctacctggccagcaacctggaaagcggagtgcctgccagatt
ttctggcagcggctctggcaccgacttcaccctgaatatccatcctgtggaagaagaggacgccgccacctactactgccagcacagta
gagagctgcccttcacattcggcagcggcaccaagctggaaatcaaagcggccgcttcttctggtggcggcggaagcgatatcaagct
ccagcaatctggcgccgagctggctagacctggcgcctctgtgaagatgagctgcaagaccagcggctacaccttcaccagatacacc
atgcactgggtcaagcagaggccaggccaaggcttggagtggatcggctacatcaaccccagccggggctacaccaactacaaccag
aagttcaaggacaaggccacactgaccaccgacaagagcagcagcacagcctacatgcagctgagcagcctgaccagcgaagatag
cgccgtgtactactgcgcccggtactacgacgatcactactgcctggattactggggccagggcacaaccctgacagtgtcatctgtgga
aggcggctctggtggcagtggtggaagcggaggatctggcggagtggacgatatccagctgacacagagccccgccatcatgagtgc
tagccctggcgagaaagtgaccatgacctgtagagccagcagcagcgtgtcctacatgaactggtatcagcagaagtccggcacaagc
cccaagcggtggatctacgatacaagcaaggtggccagcggcgtgccctacagattttctggctctggcagcggcaccagctactccct
gacaatcagcagcatggaagccgaggacgccgccacttactactgccagcagtggtctagcaaccctctgacctttggagccggcacc
aagctggaactgaagcaccaccaccatcaccactaa
SEQ ID NO: 2 (CD45xCD3 T-cell engager, protein): 
MGWSCIILFLVATATGAHSMEVKLLESGGGLVQPGGSLKLSCAASGFDFSRYWMSWV
RQAPGKGLEWIGEINPTSSTINFTPSLKDKVFISRDNAKNTLYLQMSKVRSEDTALYYC
ARGNYYRYGDAMDYWGQGTSVTVSKISGGGGSGGGGSGGGGSGGGGSGGGGSSDIV
LTQSPASLAVSLGQRATISCRASKSVSTSGYSYLHWYQQKPGQPPKLLIYLASNLESGV
PARFSGSGSGTDFTLNIHPVEEEDAATYYCQHSRELPFTFGSGTKLEIKAAASSGGGGS
DIKLQQSGAELARPGASVKMSCKTSGYTFTRYTMHWVKQRPGQGLEWIGYINPSRGY
TNYNQKFKDKATLTTDKSSSTAYMQLSSLTSEDSAVYYCARYYDDHYCLDYWGQGT
TLTVSSVEGGSGGSGGSGGSGGVDDIQLTQSPAIMSASPGEKVTMTCRASSSVSYMNW
YQQKSGTSPKRWIYDTSKVASGVPYRFSGSGSGTSYSLTISSMEAEDAATYYCQQWSS
NPLTFGAGTKLELKHHHHHH
SEQ ID NO: 3 (CD45xCD16 NK cell engager, DNA): 
atgggatggagctgtatcatcctcttcttggtagcaacagctacaggcgcgcactccatggaagtgaagctgctggaatctggcggcgga
ctggttcaacctggcggatctctgaagctgagctgtgccgccagcggcttcgacttcagcagatactggatgagctgggtccgacaggc
ccctggcaaaggactggaatggatcggcgagatcaaccccaccagcagcaccatcaacttcacccctagcctgaaggacaaggtgttc
atcagccgggacaacgccaagaacaccctgtacctgcagatgagcaaagtgcggagcgaggacacagccctgtactactgtgccaga
ggcaactactacagatacggcgacgctatggactactggggccagggaacaagcgtgaccgtgtctaagatttctggcggaggcggat
caggcggcggaggaagcggtggcggaggatcaggtggtggtggatcaggcggaggtggcagctctgatatcgtgctgacacagtct
ccagccagcctggccgtttctctgggacagagagccaccatcagctgcagagccagcaagagcgtgtccacaagcggctacagctac
ctgcactggtatcagcagaagcccggccagcctcctaagctgctgatctacctggccagcaacctggaaagcggagtgcctgccagatt
ttctggcagcggctctggcaccgacttcaccctgaatatccatcctgtggaagaagaggacgccgccacctactactgccagcacagta
gagagctgcccttcacattcggcagcggcaccaagctggaaatcaaagcggccgctggaggagggggatcaggaggcgggagcga
agtgcagttggtcgagtcagggggcgagctggtgcaacccggtggttcactccggctttcctgtgccgcctctggtctgacttttagctctt
ataacatggggtggttcaggcgagcgcccggtaaggagcgggagttcgtcgcatctatcacctggtcaggtcgggacaccttttacgcg
gacagtgtgaaaggacgatttacaatctcaagggataatgcaaagaatacggtttacttgcaaatgtctagcttgaagcctgaggacactg
ccgtctactattgcgccgctaatccctggccagtggctgcgccgcggagtggcacttattgggggcaaggtacccaggtcaccgtgagtt
ctggcggagggggatccggcggtggaagcgaggtccaacttgtagaatctgggggcggtctcgtccaacccggaaattcattgcgcct
ttcatgcgcagccagtgggtttactttcagttccttcgggatgtcatgggtaagacaagcaccaggtaaaggcttggagtgggtgtccagt
atctctggctctggaagcgatacactttacgcggattccgtgaaaggacggtttacaatttcacgggataacgctaaaacaacattgtatct
gcaaatgaactcacttcgacccgaggatacggcagtgtattattgtacaatagggggatcattgagtcgatcctcacaaggcaccctcgtt
acagtaagctcctaa
SEQ ID NO: 4 (CD45xCD16 NK cell engager, protein):

MGWSCIILFLVATATGAHSMEVKLLESGGGLVQPGGSLKLSCAASGFDFSRYWMSWV
RQAPGKGLEWIGEINPTSSTINFTPSLKDKVFISRDNAKNTLYLQMSKVRSEDTALYYC
ARGNYYRYGDAMDYWGQGTSVTVSKISGGGGSGGGGSGGGGSGGGGSGGGGSSDIV
LTQSPASLAVSLGQRATISCRASKSVSTSGYSYLHWYQQKPGQPPKLLIYLASNLESGV
PARFSGSGSGTDFTLNIHPVEEEDAATYYCQHSRELPFTFGSGTKLEIKAAAGGGGSGG
GSEVQLVESGGELVQPGGSLRLSCAASGLTFSSYNMGWFRRAPGKEREFVASITWSGR
DTFYADSVKGRFTISRDNAKNTVYLQMSSLKPEDTAVYYCAANPWPVAAPRSGTYW
GQGTQVTVSSGGGGSGGGSEVQLVESGGGLVQPGNSLRLSCAASGFTFSSFGMSWVR
QAPGKGLEWVSSISGSGSDTLYADSVKGRFTISRDNAKTTLYLQMNSLRPEDTAVYYC
TIGGSLSRSSQGTLVTVSS

## Claims

1. An antibody for use in a therapy involving the cytotoxic elimination of a target cell by engaging the target cell with an immune effector cell, the antibody comprising a first binding domain binding to an epitope on a surface protein present on the surface of the target cell and a second binding domain binding to an epitope on a surface protein present on the surface of the immune effector cell, wherein the surface protein on the surface of the target cell is also present on the surface of the immune effector cell.

2. The antibody for use in a therapy involving the cytotoxic elimination of a target cell by engaging the target cell with an immune effector cell according to claim 1, the antibody comprising a first binding domain binding to a first epitope and a second binding domain binding to a second epitope, wherein the antibody is configured to bind, with its first binding domain, to the first epitope, the first epitope being present on a first surface protein present on the surface of the target cell and on the surface of the immune effector cell, and with its second binding domain to the second epitope, the second epitope being present on a second surface protein present on the surface of the immune effector cell, but not the target cell, wherein the first and second surface protein are different from each other.

3. The antibody for use in a therapy involving the cytotoxic elimination of a target cell by engaging the target cell with an immune effector cell according to claim 2, wherein the first epitope is an epitope on CD45.

4. The antibody for use in a therapy involving the cytotoxic elimination of a target cell by engaging the target cell with an immune effector cell according to one of claims 2 or 3, wherein the second epitope is an epitope on CD3 or C16.

5. The antibody for use in a therapy involving the cytotoxic elimination of a target cell by engaging the target cell with an immune effector cell according to one of claims 2 to 4, wherein a) the immune effector cell is a T cell or a natural killer cell, b) the target cell is a malignant hematopoietic cell, c) the first epitope is an epitope on CD45, and wherein the second epitope is d₁) an epitope on CD3 in case the immune effector cell is a T cell, or d₂) the second epitope is an epitope on CD16 in case the immune effector cell is a natural killer cell.

6. The antibody for use in a therapy involving the cytotoxic elimination of a target cell by engaging the target cell with an immune effector cell according to one of claims 2 to 5, wherein the first surface protein, being present on the surface of the target cell and the immune effector cell, is present on the surface of the immune effector cell at the same or at a higher level than on the surface of the target cell.

7. The antibody for use in a therapy involving the cytotoxic elimination of a target cell by engaging the target cell with an immune effector cell according to one of the preceding claims, wherein the antibody is configured to bind to a T cell or a natural killer cell as an immune effector cell.

8. The antibody for use in a therapy involving the cytotoxic elimination of a target cell by engaging the target cell with an immune effector cell according to one of the preceding claims, wherein the antibody is configured to engage a cancer cell, preferably a malignant hematopoietic cell, as the target cell with the immune effector cell.

9. The antibody for use in a therapy involving the cytotoxic elimination of a target cell by engaging the target cell with an immune effector cell according to claim 1, wherein the surface protein on the target cell is identical to the surface protein on the immune effector cell, and wherein the antibody binds with its first binding domain to an epitope on the surface protein on the target cell, and with its second binding domain to an epitope on the identical surface protein on the immune effector cell.

10. The antibody for use in a therapy involving the cytotoxic elimination of a target cell by engaging the target cell with an immune effector cell according to claim 9, wherein the surface protein on the target cell and the surface protein on the immune effector cell are both CD3.

11. The antibody for use in a therapy involving the cytotoxic elimination of a target cell by engaging the target cell with an immune effector cell according to one of the preceding claims, wherein the antibody is a multispecific, trispecific or bispecific antibody, preferably a bispecific antibody.

12. The antibody for use in a therapy involving the cytotoxic elimination of a target cell by engaging the target cell with an immune effector cell according to one of the preceding claims, wherein the antibody is configured to being capable of activating or directing complement activity to the target cell.

13. A pharmaceutical composition or dosage form for use in a therapy involving the cytotoxic elimination of a target cell by engaging the target cell with an immune effector cell, the pharmaceutical composition comprising an antibody according to one of claims 1 to 12 and a pharmaceutically acceptable carrier.
